# EUROPEAN PATENT SPECIFICATION

(11) **EP 2 844 735 B1**
(45) Date of publication and mention of the grant of the patent: **26.06.2019**
(21) Application number: 13784638.2
(22) Date of filing: 03.05.2013
(51) Int. Cl.: A21D 2/36, C12N 1/12, A23L 27/10, A23L 27/20, A23L 5/42, A23L 5/46, A23L 29/256

(54) **FLAVOR, ODOR, AND/OR COLORANT COMPOSITIONS WITH OLEAGINOUS MICROORGANISMS AND RELATED METHODS**
GESCHMACKS-, GERUCHS- UND/ODER FÄRBEMITTELZUSAMMENSETZUNGEN MIT ÖLIGEN MIKROORGANISMEN UND ENTSPRECHENDE VERFAHREN
COMPOSITIONS DE SAVEUR, D'ODEUR ET/OU DE COLORANT AYANT DES MICROORGANISMES OLÉAGINEUX ET PROCÉDÉS ASSOCIÉS

(30) Priority: 04.05.2012 US 201261642724 P
(43) Date of publication of application: 11.03.2015
(73) Proprietor: Corbion Biotech, Inc., South San Francisco, CA 94080 (US)
(72) Inventor: Norris, Leslie, South San Francisco, CA 94080 (US)
(74) Representative: Carpmaels & Ransford LLP
(86) International application number: PCT/US2013/039446
(87) International publication number: WO 2013/166374

(56) References cited:
- US-A- 5 236 721
- US-A1- 2010 297 331
- US-A1- 2011 256 268
- US-A1- 2011 256 268
- US-A1- 2011 256 282
- US-A1- 2011 256 282
- US-B1- 6 294 207

## Description

### Technical Field

The present invention generally relates to storage and delivery of flavor, odor and/or colorant, especially for use with food and personal care products. It more specifically relates to compositions that include flavoring, odorant, and/or colorant compounds in combination with oleaginous microorganisms, and methods of making the compositions.

### Background

Algae have long been looked to as a potential source of food. While certain types of algae, primarily seaweed, do indeed provide important foodstuffs for human consumption, the promise of algae as a foodstuff has not been fully realized. Algal powders made with algae grown photosynthetically in outdoor ponds or photobioreactors are commercially available but have a deep green color (from the chlorophyll) and a strong, unpleasant taste. When formulated into food products or as nutritional supplements, these algal powders impart a visually unappealing green color to the food product or nutritional supplement and have an unpleasant fishy or seaweed flavor.

WO2010/120923, WO2010/045368 US2010/0297331, and WO2011/130578 disclose methods of making and using microalgal biomass, and especially oleaginous cell powder of *Chlorella protothecoides* as a food.

### Summary of the Invention

The present invention provides a method for producing a dried powder flavorant, odorant and/or colorant composition as defined in claim 1. Preferred features of the method are disclosed in claims 2 to 8.

The present invention also provides a dried powder flavorant, odorant and/or colorant composition as defined in claim 9 and a method of producing a food product or personal care product as defined in claim 10.

The oleaginous microorganism can be an oleaginous microalgae. For example, the oleaginous microalgae can be of the genus Chlorella or Prototheca, including, Chlorella protothecoides or Prototheca moriformis.

In specific embodiments, the oleaginous microorganism can be cultivated heterotrophically, in the dark.

In yet more specific embodiments, the excipient is one or more of cyclodextrin, gum arabic and maltodextrin.

### Brief Description of the Drawings

The features of the invention will be more readily understood by reference to the following detailed description, taken with reference to the accompanying drawing, in which:

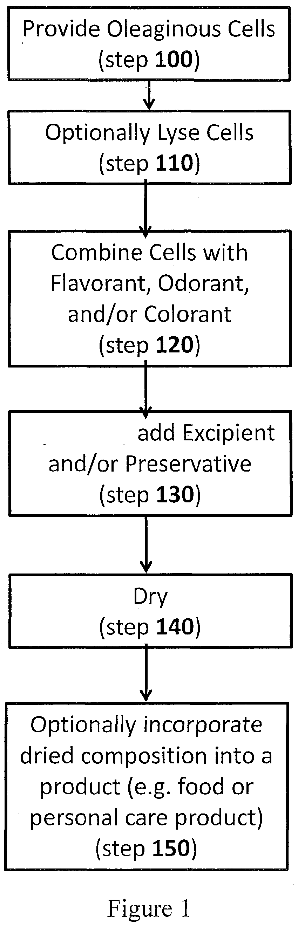

Fig. 1 shows a flow diagram in accordance with an embodiment of the present invention.

### Detailed Description of Embodiments of the Invention

### Definitions

As used in this description and the accompanying claims, the following terms shall have the meanings indicated, unless the context otherwise requires.

An "excipient" shall mean a substance other than the flavorant, odorant, colorant, or oleaginous microbe, whether inert or having functional attributes. Without limitation, excipients can function in a dried composition, or final product into which the composition is incorporated, as film formers, emulsifiers, fillers, binders, stabilizers, preservatives, antioxidants, or microbial growth inhibitors.

In connection with a cell or cells, "oleaginous" means that the cell or cells have a triglyceride content of 10 percent or more triglycerides by dry weight.

"Lysed" cells are those where the cellular wall and/or membrane have been disrupted. After lysis, the cell's contents may remain inside the disrupted cell wall or membrane or be at least partially ejected from the cell.

According to the present invention, oleaginous cells are combined with one or more of a flavorant, odorant or colorant. One or more excipients is also added. When a solvent is present in the mixture, the combined cells and ingredient(s) are then dried to form a dried composition in the form of a powder. The dried ingredient can be incorporated into a product such as a food, beverage or personal care product.

Fig. 1 shows a flow diagram of a process for making compositions according to the present invention. Oleaginous cells (step **100**) are obtained. The oleaginous cells can be cultivated using known methods, and are provided in liquid or dry form. The oleaginous cells can include, for example, 10 percent to 90 percent triglycerides, 20 percent to 80 percent triglycerides, 30 percent to 70 percent triglycerides, or 40 percent to 60 percent triglycerides.

The oleaginous cells may be obtained from a culture of oleaginous microorganisms such as oleaginous microalgae or oleaginous yeast. The cells are preferably cultivated heterotrophically, in the dark, using a fixed carbon source (i.e., one other than carbon dioxide such as glucose, sucrose, glycerol, hydrolyzed cellulosic material, etc.). The triglyceride content of the cells can be increased by cultivation under nutrient limiting conditions, especially by limiting nitrogen.

In a preferred embodiment, the oleaginous cells are microalgal cells. Non-limiting examples of oleaginous microalgae include: *Achnanthes orientalis, Agmenellum, Amphiprora hyaline, Amphora coffeiformis, Amphota coffeiformis linea, Amphora coffeiformis punctate, Amphora coffeiformis taylori, Amphora coffeiformis tenuis, Amphora delicatissima, Amphora delicatissima capitata, Amphora sp., Anabaena, Ankistrodesmus, Ankistrodesmus, Ankistrodesmus falcatus, Boekelovia hooglandii, Borodinella sp., Botryococcus braunii, Botryococcus sudeticus, Carteria, Chaetoceros gracilis, Chaetoceros muelleri, Chaetoceros muelleri subsalsum, Chaetoceros sp., Chlorella anitrata, Chlorella Antarctica, Chlorella aureoviridis, Chlorella candida, Chlorella capsulate, Chlorella desiccate, Chlorella ellipsoidea, Chlorella emersonii, Chlorella fusca, Chlorella fusca var. vacuolata, Chlorella glucotropha, Chlorella infusionum, Chlorella infusionum var. actophila, Chlorella infusionum var. auxenophila, Chlorella kessleri, Chlorella lobophora, Chlorella luteoviridis, Chlorella luteoviridis var. aureoviridis, Chlorella luteoviridis var. lutescens, Chlorella miniata, Chlorella minutissima, Chlorella mutabilis, Chlorella nocturna, Chlorella parva, Chlorella photophilia, Chlorella pringsheimii, Chlorella prototecoides (including any of UTEX strains 1806, 411, 264, 256, 255, 250, 249, 31, 29, 25), Chlorella prototecoides var. acidicola, Chlorella regularis, Chlorella regularis var. minima, Chlorella regularis var. umbricata, Chlorella reisiglii, Chlorella saccharophila, Chlorella saccharophia var. ellipsoidea, Chlorella salina, Chlorella simplex, Chlorella sorokiniana, Chlorella sp., Chlorella aphaerica, Chlorella stigmatophora, Chlorella vanniellii, Chlorella vulgaris, Chlorella vulgaris f. tertia, Chlorella vulgaris var. autotrophica, Chlorella vulgaris var. viridis, Chlorella vulgaris var. vulgaris, Chlorella vulgaris var. vulgaris f. tertia, Chlorella vulgaris var. vulgaris f. viridis, Chlorella xanthella, Chlorella zofingiensis, Chlorella trebouxioides, Chlorella vulgaris, Chlorococcum insusionum, Chlorococcum sp., Chlorogonium, Chroomonas sp., Chrysosphaera sp., Cricosphaera sp., Crypthecodinium cohnii, Cryptomonas sp., Cyclotella cryptica, Cyclotella meneghiniana, Cyclotella sp., Dunaliella sp., Dunaliella bardawil, Dunaliella bioculata, Dunaliella granulate, Dunaliella maritime, Dunaliella minuta, Dunaliella parva, Dunaliella peircei, Dunaliella primolecta, Dunaliella salina, Dunaliella terricola, Dunaliella tertiolecta, Dunaliella viridis, Eremosphaera viridis, Eremosphaera sp., Ellipsoidon sp., Euglena, Franceia sp., Fragilaria crotonensis, Fragilaria sp., Gleocapsa sp., Gloeothamnion sp., Hymenomonas sp., Isochrysis aff. Galbana, Isochrysis galbana, Lepocinclis, Micractinium, Micractinium (UTEX LB 2614), Monoraphidium minutum, Monoraphidium sp., Nanochloris sp., Nanochloropsis salina, Nanochloropsis sp., Navicula acceptata, Navicula biskanterae, Navicula pseudotenelloides, Navicula pelliculosa, Navicula saprophila, Navicula sp., Nephrochloris sp., Nephroselmis sp., Nitschia communis, Nitzschia alexandrina, Nitzschia communis, Nitzschia dissipata, Nitzschia frustulum, Nitzschia hantzschiana, Nitzschia inconspicua, Nitzschia intermedia, Nitzschia microcephala, Nitzschia pusilla, Nitzschia pusilla elliptica, Nitzschia pusilla monoensis, Nitzschia quadrangular, Nitzschia sp., Ochromonas sp., Oocystis parva Oocystis pusilla, Oocystis sp., Oscillatoria limnetica, Oscillatoria sp., Oscillatoria subbrevis, Pascheria acidophila, Pavlov asp., Phagus, Phormidium, Platymonas sp., Pleurochrysis carterae, Pleurochrysis dentate, Pleurochrysis sp., Prototheca wickerhamii, Prototheca stagnora, Prototheca portoricensis, Prototheca moriformis, Prototheca zopfii. Pyramimonas sp., Pyrobotrys, sarcinoid chrysophyte, Scenedesmus armatus, Schizochytrium, Spirogyra, Spirulina platensis, Stichococcus sp., Synechococcus sp., Tetraedron, Tetraselmis sp., Tetraselmis suecica, Thalassiosira weissflogii,* and *Viridiella fridericiana.*

In preferred embodiments, the oleaginous cells are microalgal cells from the genus *Chlorella* (e.g., *Chlorella protothecoides*) or the genus *Prototheca* (e.g., *Prototheca moriformis*).

In certain cases, the oleaginous cells are microalgae that are cultivated heterotrophically, in the dark, so as to include less than 1000 ppm, 750 ppm, 500 ppm, or 250 ppm of color generating impurities. The cells may lack a visible green color. Non-limiting examples of such impurities include chlorophyll and carotenoids. In a specific test for color, 10 mg of dried microalgae are suspended in 1 mL of water and the absorbance is measured at 500 nm or 660 nm. In specific embodiments, the absorbance so measured at 500 nm and/or 660 nm is less than or equal to 1, 0.1, 0.01, or 0.001 OD (Optical Density) unit.

In an embodiment, the oleaginous cells have between 20-115 µg/g of total carotenoids, including 20-70 µg/g lutein and preferably have less than 10 µg/g lutein.

In order to reduce unpalatable flavor and odor, cells with low levels of highly unsaturated fatty acids (fatty acids having more than three double bonds) may be used. For example, the microalgal triglyceride is less than 5%, 2.5%, 1%, or is substantially free of docosahexaenoic acid (DHA, a C22:6 fatty acid). The cells may also have 30% or greater monounsaturated fatty acids.

In general, the cells can be lacking in unpleasant taste or odor.

The cells may be mutated to further reduce the color. For example, classical mutagenesis may be used to create variability in a population of microalgae and the resulting cells screened for low color. Screening can be accomplished, for example, by: limiting dilution and culture in 96 well or 384 well plates followed by absorbance measurements in a plate reader or making chemical measurements (e.g., using chromatography, mass spectrometry or other methods). In the case of heterotrophically cultivated *Chlorella*, it has been found that mutants can be obtained by classical mutagenesis that are reduced in yellow color compared to the parent strains.

The cells are 10 to 90 percent lysed (step **110**). For example, lysis may be accomplished by mechanical, enzymatic, chemical, viral, electrical, ultrasonic, osmotic or other mechanism. Illustrative compositions of the present invention include a population of cells that is 20 to 90, or 30 to 90 percent lysed. As a result of lysis, the cells may be made more permeable to various flavorants, odorants or colorants. Lysis can be performed before or after drying of the cells. In a preferred embodiment, the cells are dried and then milled to lyse.

The 10 to 90 percent lysed cells are mixed with one or more flavorant, odorant, or colorant to form a mixture (step **120**). The flavorant, odorant, and/or colorant is a substantially pure compound, or a mixture or natural extract. These categories are not mutually exclusive. It is known in the art that flavorants can act as odorants and vice versa. Thus, embodiments of the invention include combining the oleaginous microbial cells with a substance to impart flavor or odor characteristics to a food or other substance. In addition, certain flavorants or odorants are also colored and can be used to impart flavor and/or odor together with color.

The flavorant, odorant, and/or colorant is a substantially pure compound, a mixture of substantially pure compounds, or a natural extract, that may be selected from the many known in the art. The flavorant, odorant and/or colorant may be in an aqueous or non-aqueous solvent. Where the flavorant, odorant, and/or colorant is fat soluble, it may partly or entirely partition into the triglyceride produced by the oleaginous microbe. For example, for those cells which are not lysed, and the substance is fat soluble, the substance may accumulate in lipid storage vesicles of the microbe (e.g., lipid filled plastids of an oleaginous microalgae). In this way, the fat soluble flavorant, odorant and/or colorant may be "encapsulated" in the cell or cell fragment. For those cells which are lysed and the lipid contents coat the outside of the cells, in such cases, a fat soluble substance can be carried in the lipidic coating. These various forms may also coexist when a 10 to 90 percent lysed population of oleaginous cells is used.

Non-limiting examples of flavorants or odorants include flavorants or odorants based on aldehydes, ketones, alcohols, terpenes, pyrazines, thiazols, dienals.

Examples of aldehyde flavorants include acetaldehyde (apple); benzaldehyde (cherry, almond); anisic aldehyde (licorice, anise); cinnamic aldehyde (cinnamon); citral (*e.g*., geranial, alpha citral (lemon, lime) and neral, beta citral (lemon, lime)); decanal (orange, lemon); ethyl vanillin (vanilla, cream); heliotropine, *i.e*., piperonal (vanilla, cream); vanillin (vanilla, cream); α-amyl cinnamaldehyde (spicy fruity flavors); butyraldehyde (butter, cheese); valeraldehyde (butter, cheese); citronellal (modifies, many types); decenal (citrus fruits); aldehyde C-8 (citrus fruits); aldehyde C-9 (citrus fruits); aldehyde C-12 (citrus fruits); 2-ethyl butyraldehyde (berry fruits); hexenal, *i.e*., trans-2 (berry fruits); tolyl aldehyde (cherry, almond); veratraldehyde (vanilla); 2-6-dimethyl-5-heptenal, *i.e*., Melonal™ (melon); 2,6-dimethyloctanal (green fruit); 2-dodecenal (citrus, mandarin); and combinations thereof.

Examples of ketone based flavorants or odorants include d-carvone (caraway); 1-carvone (spearmint); diacetyl (butter, cheese, "cream"); benzophenone (fruity and spicy flavors, vanilla); methyl ethyl ketone (berry fruits); maltol (berry fruits) menthone (mints), methyl amyl ketone, ethyl butyl ketone, dipropyl ketone, methyl hexyl ketone, ethyl amyl ketone (berry fruits, stone fruits); pyruvic acid (smokey, nutty flavors); acetanisole (hawthorn heliotrope); dihydrocarvone (spearmint); 2,4-dimethylacetophenone (peppermint); 1,3-diphenyl-2-propanone (almond); acetocumene (orris and basil, spicy); isojasmone (jasmine); d-isomethylionone (orris like, violet); isobutyl acetoacetate (brandy-like); zingerone (ginger); pulegone (peppermint-camphor); d-piperitone (minty); 2-nonanone (rose and tea-like); and combinations thereof.

Examples of alcohol based flavorants or odorants include anisic alcohol or p-methoxybenzyl alcohol (fruity, peach); benzyl alcohol (fruity); carvacrol or 2-p-cymenol (pungent warm odor); carveol; cinnamyl alcohol (floral odor); citronellol (rose like); decanol; dihydrocarveol (spicy, peppery); tetrahydrogeraniol or 3,7-dimethyl-1-octanol (rose odor); eugenol (clove); p-mentha-1,8dien-7-O or perillyl alcohol (floral-pine); alpha terpineol; mentha-1,5-dien-8-ol 1; mentha-1,5-dien-8-ol 2; p-cymen-8-ol; and combinations thereof.

Examples of colorants that can be used include artificial colorings such as FD&C blue No. 1, FD&C Blue No. 2, FD&C Green, No. 3, FD&C Red No. 40, FD&C Red No. 3, FD&C Yellow No. 5, FD&C Yellow, No. 5, or natural food dyes such as caramel coloring, annatto, cochineal, curcuminoids, saffron, paprika, elderberry juice, pandan, and butterfly pea.

One or more excipients comprising fillers, binders, film-formers, stabilizers or preservatives (including antioxidants and antimicrobials), are added to the mixture (step **130**). Recognizing that the oleaginous cells themselves can function to stabilize flavor, odor or color, additional stabilizing excipients (e.g., cyclodextrins) can be added as preservatives.

The composition forms an emulsion prior to drying. Film formers such as gum arabic may be added to aid in emulsion formation and/or protect the flavor/oil mixture during drying. The resulting dried product is in the form of powder. The emulsion formation may be entirely or primarily due to the oleaginous cells, or can be assisted with the addition of emulsifying agents.

Other excipients that may be used in compositions of the present invention include, without limitation: carbohydrates; gums; and, proteins. Non-limiting examples of carbohydrates include starch, modified starch, maltodextrins, and cyclodextrins (*e.g*., β-cyclodextrin). Non-limiting examples of carbohydrates include gum arabic, maltodextrin and mixtures of gum arabic and maltodextrin. Non-limiting examples of proteins include sodium caseinate, whey protein isolates, algal protein and soy protein isolates.

Non-limiting examples of optional preservatives that may be used include antimicrobial preservatives and antioxidants. Antimicrobial preservatives include, without limitation, the following: sorbic acid and its salts, benzoic acid and its salts, calcium proprionate, sodium nitrite, sodium nitrate, sulfites (e.g., sulfur dioxide, sodium bisulfite, potassium sulfite), and disodium EDTA. Examples of antioxidant preservatives include tocopherols, rosemary extract, ascorbic acid, TBHQ, propyl gallate, butylated hydroxyanisole and butylated hydroquinone.

The mixture of oleaginous cells, flavorant, odorant and/or colorant and preservative, film former, or other excipient(s) is dried. (step **140**). In a specific embodiment, heterotrophically cultivated *Chlorella* cells (partly lysed) having between 30 and 70 percent triglyceride by dry cell weight are combined with a flavorant, odorant and/or colorant together with a film forming substance such as gum arabic and dried to form fine particles.

Drying may be used at intermediate stages as well. For example, the oleaginous cells can be dried prior to combining with a solution or suspension containing the flavorant, odorant, and/or colorant, and one or more optional excipients. This mixture can then be dried. Alternatively, all the ingredients can be combined in dry form with mechanical mixing for a time and at a temperature sufficient to mix the added ingredients with the oleaginous cells.

The composition is spray-dried. Spray drying involves the atomization and spraying of the mixture into a hot chamber to form a powder. Thus, a wet mixture of flavorant, odorant and/or colorant, the oleaginous cells and one or more optional excipients in a liquid is atomized into a hot chamber. Examples of spray-driers include tower, box-form, and FilterMat™ spray driers.

In certain cases, the spray dryer has a vertical parallel flow function. Such dryers are usually capable of blowing a high volume of dry gas dehumidified to 1% relative humidity (RH) or less. Non-limiting examples of spray dryers include the micromist MD series and the hybrid granulator series manufactured by Fujisaki Electric Col, Ltd., the FSD spray dryer with internal fluid layer as manufactured by Niro Corporation, the fluid granulation spray dryer and L-8 type spray dryer manufactured by Ogawara Chemical Engineering Machine Corporation, and the DL-21 type and GB-21 type manufactured by Yamato Scientific Co., Ltd.

The spray dryer can be capable of generating liquid droplets having a mean volume diameter of approximately 0.1 µm. Oftentimes, the dryer is capable of generating liquid droplets having a mean volume diameter of approximately 0.1 µm to approximately 20 µm, 0.1 µm to approximately 10 µm, or approximately 1.0 µm to approximately 8.0 µm. When the liquid droplets are dried, a dry powder usually having a mean volume diameter of approximately 0.1 µm to approximately 15.0 µm, approximately 0.1 µm to approximately 7.0 µm, or approximately 0.7 µm to approximately 6.0 µm is prepared.

The composition can then be packaged and shipped. Alternately, the composition can be incorporated into a food product or other product (step **150**). Examples of non-food products that can be odorized or colored using the composition include personal care products such as bar soap, liquid soap, shampoos, hair conditioners, lotions, lipstick, eyeliner, mascara, rouge and other cosmetics. The compositions can also be used to color or scent plastics, air fresheners or other items.

Compositions of the present invention can be included in a variety of different food types. Non-limiting examples of such food types include: baked goods, such as bread, cakes, cookies, crackers and cereals; fried goods, such as chips; frozen goods, such as ice cream, sherbets, and popsicles; prepared sauces, such as pasta sauces, cheese-based sauce, and milk-based sauces; and, flavor packets, as used in boxed grains, pastas, stuffings and potatoes.

When flavorant compositions according to embodiments of the present invention are incorporated into food products, they can protect against flavor loss, flavor character change (fresh flavor changing to cooked flavor) and/or carry more flavor. For example, flavorant, odorant and/or colorant dried in the above-described compositions can deliver the flavor, odor or color to the finished food without loss in a hot-fill or retort process. This flavor protection and/or enhancement may be measured using a variety of methods. For instance, gas chromatography (*i.e*., GC), gas chromatography/mass spectrometry (*i.e*., GC/MS), or high performance liquid chromatography (*i.e*., HPLC) can be used to identify and quantify one or more specific flavorants, or their degradants, in a food product. Human sensory testing can also be used to determine flavor qualities of a food product. *See*, for example, ASTM 1627-11.

In an embodiment, heating a food product containing one or more compositions according to various embodiments of the present invention to approximately 90 °C to 190 °C at a pressure between 0.1 lb/in² and 45 lb/in², or 95 °C to 130 °C at a pressure between 15 lb/in² and 35 lb/in², for a period of approximately one, two, three, four or five minutes typically does not result in the degradation of more than 25 percent of a particular flavorant, odorant or colorant. The heating does not result in the degradation of more than 20 percent, 15 percent, or 10 percent of the flavorant. In certain cases, it does not result in the degradation of more than 7.5 percent, 5.0 percent, 4.0 percent, 3.0 percent or 1.0 percent of the flavorant.

### Example: Vanilla Cake.

A dry powder of *Chlorella protothecoides* cells having approximately 50% lipid by dry cell weight and about 90% lysed was produced. A mixture of *Chlorella protothecoides* powder (5%), water (65%), vanilla flavoring (10%), and gum arabic (20%), all percents by weight, was mixed in a shear mixer and dried using a form of spray drying resulting in dried flakes, The flakes were incorporated into a yellow cake at a concentration of 0.1% by weight flakes. A control cake was prepared using vanilla flavoring without the *Chlorella* flakes. Based on taste tests, the cake made with the *Chlorella* flakes was higher in vanillin character when compared to the control cake.

## Claims

1. A method for producing a dried powder flavorant, odorant and/or colorant composition, the method comprising:
a) providing oleaginous microalgal cells;
b) optionally lysing said cells;
c) combining said optionally lysed cells with one or more of a flavorant, an odorant or a colorant, wherein the or each flavorant, odorant and/or colorant consists of a substantially pure compound, a mixture of substantially pure compounds or a natural extract, and adding one or more excipients, wherein the one or more excipients comprises a filler, binder, film-former, stabilizer or preservative, to provide a wet mixture in the form of an emulsion; and
d) spray drying the wet mixture to produce a dried powder flavorant, odorant and/or colorant composition wherein the cells are 10-90 percent lysed.

2. A method according to claim 1, wherein the cells in the dried powder composition are 30-70 or 40-60 percent lysed.

3. A method according to claim 1 or claim 2, wherein the oleaginous cells provided in step a) comprise 10-90, 20-80, 30-70 or 40-60 percent triglyceride by dry cell weight.

4. A method according to any one of claims 1 to 3, wherein the oleaginous cells provided in step a) have one or more of the following properties:
(a) less than 2.5% DHA;
(b) less than 2 ppm chlorophyll;
(c) less than 500 ppm of color generating impurities; or
(d) lacking in an unpleasant odor.

5. A method according to any one of claims 1 to 4, wherein the microalgal cells are from the genus *Chlorella* or the genus *Prototheca.*

6. A method according to claim 5, wherein the microalgal cells are of the species *Chlorella protothecoides* or *Prototheca moriformis.*

7. A method according to claim 6, wherein the microalgal cells are of the species *Prototheca moriformis.*

8. A method according to any one of claims 1 to 7, wherein the flavorant, odorant and or colorant is/are an aldehyde-, ketone-, alcohol-, terpene-, pyrazine-, thiazol- or dienal-based flavorant or odorant, an artificial coloring and/or a natural food dye.

9. A dried powder flavorant, odorant and/or colorant composition obtainable by the method of any one of claims 1 to 8.

10. A method of producing a food product or personal care product, preferably wherein the food product is selected from the group consisting of: baked goods; fried goods; frozen goods; prepared sauces; and flavor packets wherein a dried powder flavorant, odorant and/or colorant composition of claim 9 is incorporated into said food product or personal care product.

## Patentansprüche

1. Verfahren für die Herstellung einer getrockneten Pulvergeschmacks-, -geruchs- und/oderfärbemittelzusammensetzung, wobei das Verfahren Folgendes umfasst:
a) Bereitstellen öliger Mikroalgenzellen;
b) wahlweise Lysieren der Zellen;
c) Kombinieren der wahlweise lysierten Zellen mit einem oder mehreren von einem Geschmacksmittel, einem Geruchsmittel oder einem Färbemittel, wobei das oder jedes Geschmacksmittel, Geruchsmittel und/oder Färbemittel aus einer im Wesentlichen reinen Verbindung, einer Mischung von im wesentlichen reinen Verbindungen oder einem natürlichen Extrakt besteht, und Zusetzen eines oder mehrerer Trägerstoffe, wobei der eine oder die mehreren Trägerstoffe einen Füllstoff, ein Bindemittel, einen Filmbildner, Stabilisator oder ein Konservierungsmittel umfasst, um eine nasse Mischung in Form einer Emulsion bereitzustellen; und
d) Sprühtrocknen der nassen Mischung, um eine getrocknete Pulvergeschmacks-, -geruchs- und/oder -färbemittelzusammensetzung herzustellen, wobei die Zellen 10-90 Prozent lysiert sind.

2. Verfahren nach Anspruch 1, wobei die Zellen in der getrockneten Pulverzusammensetzung 30-70 oder 40-60 Prozent lysiert sind.

3. Verfahren nach Anspruch 1 oder Anspruch 2, wobei die in Schritt a) bereitgestellten öligen Zellen 10-90, 20-80, 30-70 oder 40-60 Prozent Triglycerid, auf das Zelltrockengewicht bezogen, umfassen.

4. Verfahren nach irgendeinem der Ansprüche 1 bis 3, wobei die in Schritt a) bereitgestellten öligen Zellen eine oder mehrere der folgenden Eigenschaften aufweisen:
(a) weniger als 2,5 % DHA;
(b) weniger als 2 ppm Chlorophyll;
(c) weniger als 500 ppm farberzeugende Verunreinigungen; oder
(d) Fehlen eines unangenehmen Geruchs.

5. Verfahren nach irgendeinem der Ansprüche 1 bis 4, wobei die Mikroalgenzellen zu der Gattung *Chlorella* oder der Gattung *Prototheca* gehören.

6. Verfahren nach Anspruch 5, wobei die Mikroalgenzellen zu der Spezies *Chlorella protothecoides* oder *Prototheca moriformis* gehören.

7. Verfahren nach Anspruch 6, wobei die Mikroalgenzellen zu der Spezies *Prototheca moriformis* gehören.

8. Verfahren nach irgendeinem der Ansprüche 1 bis 7, wobei das Geschmacksmittel, Geruchsmittel und/oder Färbemittel ein Geschmacksmittel oder Geruchsmittel auf der Basis von Aldehyd, Keton, Alkohol, Terpen, Pyrazin, Thiazol oder Dienal, ein künstliches Farbmittel und/oder ein natürlicher Nahrungsmittelfarbstoff ist.

9. Getrocknete Pulvergeschmacks-, -geruchs- und/oder - färbemittelzusammensetzung, die durch das Verfahren nach irgendeinem der Ansprüche 1 bis 8 erhältlich ist.

10. Verfahren für die Herstellung eines Nahrungsmittelprodukts oder persönlichen Pflegeprodukts, wobei das Nahrungsmittelprodukt aus der Gruppe ausgewählt wird bestehend aus: Backwaren, gebratenen Waren, Tiefkühlgütern, Fertigsaucen und geschmackgebenden Packungen, wobei eine Pulvergeschmacks-, -geruchs- und/oderfärbemittelzusammensetzung nach Anspruch 9 in das Nahrungsmittelprodukt oder persönliche Pflegeprodukt integriert ist.

## Revendications

1. Méthode de production d'une composition aromatisante, odorante et/ou colorante en poudre séchée, la méthode comprenant :
a) la fourniture de cellules de microalgues oléagineuses ;
b) éventuellement la lyse desdites cellules ;
c) la combinaison desdites cellules éventuellement lysées avec un ou plusieurs parmi un arôme, une substance odorante ou un colorant, où le ou chacun parmi l'arôme, la substance odorante et/ou le colorant est constitué d'un composé sensiblement pur, d'un mélange de composés sensiblement purs ou d'un extrait naturel, et l'addition d'un ou plusieurs excipients, où les un ou plusieurs excipients comprennent une charge, un liant, un agent filmogène, un stabilisant ou un conservateur, afin de fournir un mélange humide sous la forme d'une émulsion ; et
d) le séchage par pulvérisation du mélange humide afin de produire une composition aromatisante, odorante et/ou colorante en poudre séchée où les cellules sont lysées à 10-90%.

2. Méthode selon la revendication 1, dans laquelle les cellules dans la composition de poudre séchée sont lysées à 30-70 ou 40-60%.

3. Méthode selon la revendication 1 ou la revendication 2, dans laquelle les cellules oléagineuses fournies dans l'étape a) comprennent 10-90, 20-80, 30-70 ou 40-60% de triglycérides en poids de cellules sèches.

4. Méthode selon l'une quelconque des revendications 1 à 3, dans laquelle les cellules oléagineuses fournies dans l'étape a) possèdent une ou plusieurs parmi les propriétés suivantes :
(a) moins de 2,5% de DHA ;
(b) moins de 2 ppm de chlorophylle ;
(c) moins de 500 ppm d'impuretés génératrices de couleur ; ou
(d) sont dépourvues d'une odeur désagréable.

5. Méthode selon l'une quelconque des revendications 1 à 4, dans laquelle les cellules de microalgues sont issues du genre *Chlorella* ou du genre *Prototheca.*

6. Méthode selon la revendication 5, dans laquelle les cellules de microalgues sont issues de l'espèce *Chlorella protothecoides* ou *Prototheca moriformis.*

7. Méthode selon la revendication 6, dans laquelle les cellules de microalgues sont issues de l'espèce *Prototheca moriformis.*

8. Méthode selon l'une quelconque des revendications 1 à 7, dans laquelle l'arôme, la substance odorante et/ou le colorant est ou sont constitués d'un arôme ou d'une substance odorante à base d'aldéhyde, de cétone, d'alcool, de terpène, de pyrazine, de thiazol ou de diénal, d'un colorant artificiel et/ou d'un colorant alimentaire naturel.

9. Composition aromatisante, odorante et/ou colorante en poudre séchée, pouvant être obtenue par la méthode selon l'une quelconque des revendications 1 à 8.

10. Méthode de production d'un produit alimentaire ou d'un produit de soin personnel, préférablement où le produit alimentaire est choisi dans le groupe constitué par les marchandises cuites, les marchandises frites ; les marchandises congelées ; les sauces préparées ; et les sachets aromatisants, où une composition aromatisante, odorante et/ou colorante en poudre séchée selon la revendication 9 est incorporée dans ledit produit alimentaire ou ledit produit de soin personnel.
